# EUROPÄISCHE PATENTSCHRIFT

(11) **EP 1 133 468 B1**
(45) Veröffentlichungstag und Bekanntmachung des Hinweises auf die Patenterteilung: **20.08.2003**
(21) Anmeldenummer: 99959338.7
(22) Anmeldetag: 25.11.1999
(51) Int. Cl.: C07C 277/08, C07C 279/36

(54) **VERFAHREN ZUR HERSTELLUNG VON N-ALKYL-N'-NITROGUANIDIN**
METHOD OF PRODUCING N-ALKYL-N'-NITROGUANIDINE
PROCEDE POUR PREPARER DE LA N-ALKYLE-N'-NITROGUANIDINE

(30) Priorität: 25.11.1998 DE 19854511; 29.09.1999 DE 19946321
(43) Veröffentlichungstag der Anmeldung: 19.09.2001
(73) Patentinhaber: Dynamit Nobel GmbH Explosivstoff- und Systemtechnik, 53840 Troisdorf (DE)
(72) Erfinder: PABST, Winfried, D-53797 Lohmar (DE); SCHIRRA, Rainer, D-53797 Lohmar (DE)
(74) Vertreter: Uppena, Franz, Dr.
(86) Internationale Anmeldenummer: EP9909142
(87) Internationale Veröffentlichungsnummer: WO00031025

(56) Entgegenhaltungen:
- EP-A- 0 798 293
- BERND EISTERT ET AL.: "Weitere Versuche mit Naphthochinon-(1.4) und dessn Halogen-, Alkyl- und Phenyl-Derivaten" JUSTUS LIEBIGS ANNALEN DER CHEMIE, Bd. 750, 1971, Seiten 1-11, XP002130795 WEINHEIM DE
- MCKAY A F ET AL: "PREPARATION AND PROPERTIES OF N-METHYL-N-NITROSO-N'-NITROGUANIDINE" JOURNAL OF THE AMERICAN CHEMICAL SOCIETY,US,AMERICAN CHEMICAL SOCIETY, WASHINGTON, DC, Bd. 69, 1. Dezember 1947 (1947-12-01), Seiten 3028-3030, XP002033022 ISSN: 0002-7863 in der Anmeldung erwähnt

## Beschreibung

Die Erfindung betrifft ein neues Verfahren zur Herstellung von N-Alkyl-N'-nitroguanidin.

In JACS 76,1877 (1954) wird ein Verfahren zur Herstellung von N-Methyl-N'-nitroguanidin beschrieben, bei dem 5-Methylisothiuroniumsulfat in üblicher Weise nitriert und anschließend in einer zweiten Reaktionsstufe die Methylmercaptogruppe gegen Methylamin substituiert wird. Nachteilig daran ist, daß es sich um eine zweistufige Umsetzung handelt. Die Ausbeuten in beiden Stufen sind zwar recht gut, die Abspaltung von Methylmercaptan, vor allem bei einer Durchführung im großtechnischen Maßstab, wirft verfahrenstechnisch Probleme auf.

Bekannt ist weiterhin, daß N-Methyl-N'-nitroguanidin dadurch erhalten werden kann, daß eine alkalische Lösung (Kaliumhydroxid) von Nitroguanidin mit Methylamin-Hydrochlorid bei 60 °C umgesetzt wird (JACS 69,3028 (1947)). Zur Entfernung anorganischer Verunreinigungen ist es bei diesem Verfahren notwendig, ein bis zwei Umkristallisationen vorzusehen. Ausbeuteverluste sind dabei nicht zu vermeiden.

In der DE-A-19 61 16 54 wird ein Verfahren zur Herstellung von N-Methyl-N'nitroguanidin beschrieben, bei dem Nitroguanidin mit wäßriger Methylaminlösung bei Temperaturen zwischen 0 °C und 40 °C umgesetzt wird. Nachteilig an diesem Verfahren sind die langen Reaktionszeiten von 24 Stunden, die sich durch die niedrigen Reaktionstemperaturen ergeben.

Aufgabe der vorliegenden Erfindung war es, ein Verfahren bereitzustellen, das in der Lage ist, N-Alkyl-N'-nitroguanidin in guten Ausbeuten unter möglichst selektiven Bedingungen bei deutlich reduzierten Reaktionszeiten herzustellen.

Überraschenderweise konnte diese Aufgabe durch ein Verfahren mit den Merkmalen des Hauptanspruchs gelöst werden. Vorzugsweise Ausgestaltungen werden durch die Unteransprüche charakterisiert.

Erfindungsgemäß wird in eine Suspension aus Nitroguanidin bei Temperaturen > 60 °C, vorzugsweise bei Temperaturen von 60 bis 90 °C, besonders bevorzugt bei Temperaturen von 60 bis 75 °C, eine Alkylaminlösung zudosiert. Hierbei ist unter Alkylamin ein verzweigtes oder unverzweigtes Niederalkylamin mit 1 bis 6 C-Atomen, Benzylamin oder Cyclohexylamin, vorzugsweise ein Hexyl-, Pentyl-, tert.-Butyl, Butyl-, Isopropyl-, Propyl-, Ethyl oder Methylamin, besonders bevorzugt Methylamin zu verstehen. Vorzugsweise wird eine 40 %ige Methylaminlösung zudosiert. Dabei wird das Gleichgewicht in Richtung des Reaktionsendprodukts verschoben, indem der entstehende Ammoniak entfernt, vorzugsweise ausgetrieben wird. Vorteilhaft geschieht dies erfindungsgemäß mittels Durchleiten von Luft, Stickstoff, CO₂ oder anderen geeigneten Gasen. Abgefangen werden kann der entstehende Ammoniak auch durch die Zugabe von Säure, beispielsweise Salpetersäure, oder Trockeneis (CO₂). Der pH-Wert sollte dabei möglichst ≥ 9 sein. Durch diese Reaktionsführung wird es ermöglicht, daß das Alkylamin unmittelbar abreagiert. Auf diese Weise ist überraschenderweise eine Reaktionsführung bei wesentlich höheren Temperaturen möglich. Das Ergebnis ist eine im Vergleich zur DE-A-196 11 654 wesentliche Verkürzung der Reaktionsdauer. Bereits nach 15 bis 20 Minuten ist die Reaktion abgeschlossen. Das in hoher Reinheit entstandene N-Alkyl-N'-nitroguanidin, beispielsweise das N-Methyl-N'-nitroguanidin wird nach dem Abkühlen abfiltriert, gewaschen und getrocknet. Um zu verhindern, daß sich beim Abkühlprozeß Nadeln bilden, können kristallbeeinflussende Additive, beispielsweise Polyvinylalkohol zugesetzt werden.

Als Ausgangsstoff kann alternativ anstelle des freien Nitroguanidins auch das Nitroguanidin-Nitrat eingesetzt werden. Die dabei durch Hydrolyse entstehende Säure wird vorzugsweise vor der Alkylaminzugabe auf einen pH-Wert ≥ 9 neutralisiert.

Neben dem diskontinuierlichen Verfahren ist es auch möglich, das N-Alkyl-N'nitroguanidin, vorzugsweise das N-Methyl-N'-nitroguanidin in einem kontinuierlichen Verfahren herzustellen. Dabei wird in einem ersten Kessel zu einer gerührten Nitroguanidinsuspension wäßrige Alkylaminlösung, wobei die Alkylamine wie oben definiert sind, vorzugsweise Methylaminlösung bei Temperaturen > 60 °C zugegeben. Im nachfolgenden Kessel läßt man die Komponenten ausreagieren, bevor die Reaktionslösung in einem dritten Kessel abgekühlt und das entstehende N-Alkyl-N'-nitroguanidin, vorzugsweise das N-Methyl-N'-nitroguanidin abfiltriert wird. Auch bei diesem kontinuierlichen Verfahren wird das Gleichgewicht in Richtung des Reaktionsendprodukts verschoben, indem der entstehende Ammoniak wie oben beschrieben entfernt wird. Sowohl das beim diskontinuierlichen, als auch das beim kontinuierlichen Verfahren entstehende N-Alkyl-N'-nitroguanidin kann gegebenenfalls umkristallisiert werden. Der Schmelzpunkt des auf diese Weise erfindungsgemäß erhaltenen N-Methyl-N'-nitroguanidins beträgt 159 bis 161 °C.

Die Ausgangsstoffe Nitroguanidin und die eingesetzten Alkylamine, beispielsweise das Methylamin sind allgemein bekannte Verbindungen der organischen Chemie. Das erfindungsgemäße Verfahren wird in der Regel in wässriger Suspension durchgeführt. Es ist aber auch möglich, in organisch/wässrigen Systemen zu arbeiten, wobei alle üblichen mit Wasser mischbaren organischen Lösungsmittel eingesetzt werden können. Beispielhaft genannt seien Ketone, wie Aceton, Methyl-ethyl-keton oder Methyl-isobutyl-keton, Nitrile, wie Acetonitril oder Propionitril sowie Alkohole, wie Methanol oder Ethanol.

Die nachfolgenden Beispiele sollen das erfindungsgemäße Verfahren erläutern, ohne es jedoch einzuschränken:

### Beispiel 1 (diskontinuierliches Verfahren):

35 g Nitroguanidin wurden in 200 ml Wasser suspendiert und auf 70°C erhitzt. In diese Suspension dosierte man innerhalb von 5 Minuten 40 g 40 %ige wässrige Methylaminlösung. Reaktionszeit ca. 15 Minuten. Zur Entfernung des entstehenden Ammoniaks wurde Luft oder Stickstoff durch einen Begasungsrührer in die Reaktionslösung eingeblasen. Nach dem Abkühlen auf ca. 5°C wurde das N-Methyl-N'-Nitroguanidin abfiltriert, mit Eiswasser gewaschen und getrocknet. Die Ausbeute betrug 65 % der Theorie. Der Schmelzpunkt des N-Methyl-N'-Nitroguanidin lag bei 162 °C. Die Reinheit betrug > 98 %.

### Beispiel 2 (diskontinuierliches Verfahren):

35 g Nitroguanidin wurden in 200 ml Mutterlauge aus Beispiel 1 suspendiert, der pH-Wert wird auf etwa 9 eingestellt und die Reaktionslösung auf 70°C erhitzt. In diese Suspension dosierte man innerhalb von 5 Minuten 40 g 40 %ige wässrige Methylaminlösung. Reaktionszeit ca. 15 Minuten. Zur Entfernung des entstehenden Ammoniaks wurde Luft oder Stickstoff durch einen Begasungsrührer in die Reaktionslösung eingeblasen. Nach dem Abkühlen auf ca. 5 °C wurde das N-Methyl-N'-Nitroguanidin abfiltriert, mit Eiswasser gewaschen und getrocknet. Die Ausbeute betrug 85 % der Theorie. Der Schmelzpunkt des N-Methyl-N'-Nitroguanidin lag bei 162 °C. Die Reinheit betrug > 98 %.

### Beispiel 3 (diskontinuierliches Verfahren):

35 g Nitroguanidin wurden in 200 ml Wasser suspendiert und auf 70°C erhitzt. In diese Suspension dosierte man innerhalb von 5 Minuten 40 g 40 %ige wässrige Methylaminlösung. Reaktionszeit ca. 15 Minuten. Während dieser Zeit wurde der pH-Wert durch Zugabe von Salpetersäure zwischen 9 und 10 gehalten. Nach dem Abkühlen auf ca. 5 °C wurde das N-Methyl-N'-Nitroguanidin abfiltriert, mit Eiswasser gewaschen und getrocknet. Die Ausbeute betrug 70% der Theorie. Der Schmelzpunkt des N-Methyl-N'-Nitroguanidin betrug 162 °C. Die Reinheit betrug > 98 %.

### Beispiel 4 (Kontinuierliches Verfahren):

In einer Kaskade aus 3 Rührgefäßen wurde im ersten Kessel Nitroguanidin, Wasser und 40 g Methylaminlösung (40 %ig) im Verhältnis 1:24:1,15 bei 70 °C gerührt. Im 2. Gefäß wurde die Lösung bereits klar und war ausreagiert. Im nachfolgenden 3. Gefäß wurde auf ca. 5 °C gekühlt, so daß das N-Methyl-N'-Nitroguanidin ausfiel. Zur Entfernung des entstehenden Ammonikas wurde in alle Gefäße Luft oder Stickstoff eingeblasen. Nach dem Abkühlen wurde das N-Methyl-N'-Nitroguanidin abfiltriert, mit Eiswasser gewaschen und getrocknet. Die Ausbeute betrug 70 % der Theorie. Der Schmelzpunkt des N-Methyl-N'-Nitroguanidin betrug 162 °C. Die Reinheit betrug > 98 %.

### Beispiel 5 (Kontinuierliches Verfahren):

Als Ausgangsprodukt wurde Nitroguanidinnitrat eingesetzt. In eine Kaskade aus 4 Rührgefäßen wurde in das 1. Gefäß ohne Heizung Nitroguanidinnitrat, Wasser und Methylaminlösung eindosiert. Das 2. Gefäß wurde auf 70 °C erhitzt. Der pH-Wert wurde durch Überschußdosierung von Methylaminlösung oder durch zusätzliche Ammoniakzugabe im ersten Gefäß auf einen Wert von 9,5 eingestellt. Im 3. Gefäß erfolgte die Nachreaktion bei 60 bis 70 °C. Das 4. Gefäß wurde abgekühlt, so daß das N-Methyl-N'-Nitroguanidin abfiltriert, gewaschen und getrocknet werden konnte. Die Ausbeute betrug 67 % der Theorie. Der Schmelzpunkt des N-Methyl-N'-Nitroguanidin betrug 160 °C. Die Reinheit betrug > 98 %.

## Patentansprüche

1. Verfahren zur Herstellung von N-Alkyl-N'-Nitroguanidin, **dadurch gekennzeichnet, daß** in eine Suspension aus Nitroguanidin oder Nitroguanidinnitrat bei Temperaturen von > 60 °C Alkylaminlösung zudosiert wird und der entstehende Ammoniak entfernt wird.

2. Verfahren zur Herstellung von N-Alkyl-N'-Nitroguanidin gemäß Anspruch 1, **dadurch gekennzeichnet, daß** der entstehende Ammoniak mittels Durchleiten von Luft, Stickstoff, CO₂ oder anderen geeigneten Gasen entfernt wird.

3. Verfahren zur Herstellung von N-Alkyl-N'-Nitroguanidin gemäß Anspruch 1 oder 3, **dadurch gekennzeichnet, daß** der entstehende Ammoniak durch Neutralisieren mit Säure abgefangen wird.

4. Verfahren zur Herstellung von N-Alkyl-N'-Nitroguanidin gemäß einem oder mehreren der Ansprüche 1 bis 3, **dadurch gekennzeichnet, daß** mindestens ein kristallbeeinflussendes Additiv, vorzugsweise Polyvinylalkohol zugesetzt wird.

5. Verfahren zur Herstellung von N-Alkyl-N'-Nitroguanidin gemäß einem oder mehreren der Ansprüche 1 bis 4, **dadurch gekennzeichnet, daß** Wasser oder organisch/wässrige Suspensionsmittel eingesetzt werden.

6. Verfahren zur Herstellung von N-Alkyl-N'-Nitroguanidin gemäß einem oder mehreren der Ansprüche 1 bis 5, **dadurch gekennzeichnet, daß** als mit Wasser mischbare organische Lösungsmittel Ketone, vorzugsweise Aceton, Methyl-ethyl-keton oder Methyl-isobutyl-keton; Nitrile, vorzugsweise Acetonitril oder Propionitril oder Alkohole, vorzugsweise Methanol oder Ethanol eingesetzt werden.

7. Verfahren zur Herstellung von N-Alkyl-N'-Nitroguanidin gemäß einem oder mehreren der Ansprüche 1 bis 6, **dadurch gekennzeichnet, daß** als Alkylamin ein verzweigtes oder unverzweigtes Niederalkylamin, wobei unter Niederalkyl C₁- bis C₆-Alkyl zu verstehen ist, Benzylamin oder Cyclohexylamin eingesetzt wird.

8. Verfahren zur Herstellung von N-Alkyl-N'-Nitroguanidin gemäß einem oder mehreren der Ansprüche 1 bis 6, **dadurch gekennzeichnet, daß** als Alkylamin Hexyl-, Pentyl-, tert.-Butyl, Butyl-, Isopropyl-, Propyl-, Ethyl oder Methylamin, besonders bevorzugt Methylamin eingesetzt wird.

## Claims

1. Method for the preparation of N-alkyl-N'-nitroguanidine, **characterised in that** metered doses of alkylamine solution are added to a suspension of nitroguanidine or nitroguanidine nitrate at temperatures of > 60°C, and the ammonia that is formed is removed.

2. Method for the preparation of N-alkyl-N'-nitroguanidine according to claim 1, **characterised in that** the ammonia that is formed is removed by passing through air, nitrogen, CO₂ or other suitable gases.

3. Method for the preparation of N-alkyl-N'-nitroguanidine according to claim 1 or 2, **characterised in that** the ammonia that is formed is captured by neutralization with acid.

4. Method for the preparation of N-alkyl-N'-nitroguanidine according to one or more of claims 1 to 3, **characterised in that** at least one crystal-influencing additive, preferably polyvinyl alcohol, is added.

5. Method for the preparation of N-alkyl-N'-nitroguanidine according to one or more of claims 1 to 4, **characterised in that** water or organic/aqueous suspension agents are used.

6. Method for the preparation of N-alkyl-N'-nitroguanidine according to one or more of claims 1 to 5, **characterised in that** ketones, preferably acetone, methyl-ethyl-ketone or methyl-isobutyl-ketone; nitriles, preferably acetonitrile or propionitrile, or alcohols, preferably methanol or ethanol, are used as organic solvents that are mixable with water.

7. Method for the preparation of N-alkyl-N'-nitroguanidine according to one or more of claims 1 to 6, **characterised in that** a branched or non-branched low alkylamine, with C₁- to C₆-alkyl being understood by low alkyl, benzylamine or cyclohexylamine is used as the alkylamine.

8. Method for the preparation of N-alkyl-N'-nitroguanidine according to one or more of claims 1 to 6, **characterised in that** hexyl-, pentyl-, tert.-butyl, butyl-, isopropyl-, propyl-, ethyl or methylamine, particularly preferably methylamine, is used as the alkylamine.

## Revendications

1. Procédé de préparation de N-alkyl-N'-nitroguànidine, **caractérisé par le fait que** l'on ajoute progressivement et de manière contrôlée, à une température supérieure à 60 ° C, une solution d'une alkylamine à une suspension de nitroguanidine ou de nitrate de nitroguanidine et que l'on élimine l'ammoniac formé.

2. Procédé de préparation de N-alkyl-N'-nitroguanidine selon la revendication 1, **caractérisé par le fait que** l'ammoniac formé est éliminé par le passage d'un flux d'air, d'azote, de CO₂ ou d'autres gaz appropriés.

3. Procédé de préparation de N-alkyl-N'-nitroguanidine selon la revendication 1 ou 2, **caractérisé par le fait que** l'ammoniac formé est retenu par neutralisation avec un acide.

4. Procédé de préparation de N-alkyl-N'-nitroguanidine selon une ou plusieurs des revendications 1 à 3, **caractérisé par le fait que** l'on ajoute au moins un additif modifiant la cristallisation, de préférence du poly(alcool vinylique).

5. Procédé de préparation de N-alkyl-N'nitroguanidine selon une ou plusieurs des revendications 1 à 4, **caractérisé par le fait que** l'on utilise de l'eau ou un milieu de suspension organoaqueux.

6. Procédé de préparation de N-alkyl-N'-nitroguanidine selon une ou plusieurs des revendications 1 à 5, **caractérisé par le fait que** l'on utilise, en tant que solvants organiques miscibles avec l'eau, des cétones, de préférence de l'acétone, de la méthyléthylcétone ou de la méthylisobutylcétone, des nitriles, de préférence de l'acétonitrile ou du propionitrile, ou des alcools, de préférence du méthanol ou de l'éthanol.

7. Procédé de préparation de N-alkyl-N'-nitroguanidine selon une ou plusieurs des revendications 1 à 6, **caractérisé par le fait que** l'on utilise en tant qu'alkylamine une alkylamine inférieure, linéaire ou ramifiée, de la benzylamine ou de la. cyclohexylamine, le terme « alkylamine inférieure » englobant les alkylamines en C₁₋₆,.

8. Procédé de préparation de N-alkyl-N'-nitroguanidine selon une ou plusieurs des revendications 1 à 6, **caractérisé par le fait que** l'on utilise en tant qu'alkylamine de l'hexylamine, de la pentylamine, de la tert-butylamine, de la butylamine, de l'isopropylamine, de la propylamine, de l'éthylamine ou de la méthylamine, de préférence de la méthylamine.
